# FASCICULE DE BREVET EUROPEEN

(11) **EP 0 794 239 B1**
(45) Date de publication et mention de la délivrance du brevet: **24.10.2001**
(21) Numéro de dépôt: 97400463.2
(22) Date de dépôt: 28.02.1997
(51) Int. Cl.: C10G 2/00, C07C 1/06

(54) **Conversion du gaz de synthèse en hydrocarbures en présence d'une phase liquide**
Umwandlung von Synthesegas in Kohlenwasserstoffe in Anwesenheit einer flüssigen Fase
Conversion of synthesis gas into hydrocarbons in the presence of a liquid phase

(30) Priorité: 08.03.1996 FR 9602911
(43) Date de publication de la demande: 10.09.1997
(73) Titulaire: INSTITUT FRANCAIS DU PETROLE, 92502 Rueil-Malmaison (FR); AGIP PETROLI S.p.A., 00142 Roma (IT)
(72) Inventeur: Euzen, Jean-Paul, 69570 Dardilly (FR); Harter, Isabelle, 69003 Lyon (FR); Chaumette, Patrick, 78380 Bougival (FR)

(56) Documents cités:
- EP-A- 0 188 304
- EP-A- 0 212 202
- DE-C- 897 549
- GB-A- 728 543
- NL-A- 7 708 307

## Description

La présente invention concerne un procédé de synthèse d'hydrocarbures essentiellement C₅⁺ (c'est-à-dire d'hydrocarbures comportant au moins 5 atomes de carbone par molécule), utilisables en tant que carburant ou combustible liquide, à partir de gaz de synthèse.

Le gaz de synthèse est un mélange CO-(CO₂)-H₂, c'est-à-dire un mélange CO-H₂ de monoxyde de carbone (CO) et d'hydrogène (H₂) comprenant éventuellement du dioxyde de carbone (CO₂). La réaction de synthèse d'hydrocarbures à partir de gaz de synthèse, opérée généralement à une température comprise entre 150 et 350°C et sous pression, est connue sous le nom de synthèse Fischer-Tropsch. Les catalyseurs habituellement utilisés pour la transformation de mélanges CO-(CO₂)-H₂ en hydrocarbures liquides ou gazeux comprennent généralement au moins un métal du groupe VIII tel que le fer, le ruthénium, le cobalt ou le nickel.

Les produits préparés par synthèse Fischer-Tropsch en présence de ces catalyseurs métalliques présentent une distribution très large en terme de poids moléculaire. La réaction que l'on cherche à réaliser dans le cadre de la présente demande est la réaction de synthèse d'hydrocarbures essentiellement C₅⁺ à partir de gaz de synthèse.

La synthèse Fischer-Tropsch est une réaction très exothermique. Si bien que lorsque le procédé pour la mise en oeuvre de ladite réaction de synthèse est opéré en phase gazeuse et avec un catalyseur en lit fixe, la conversion du monoxyde de carbone doit être limitée en deçà de 85%, afin d'éviter les instabilités thermiques dans le lit catalytique, ce qui oblige à séparer et recycler le gaz de synthèse non converti.

Un autre procédé qui a été envisagé pour la mise en oeuvre de la réaction consiste à opérer en présence d'une phase liquide et avec un catalyseur en suspension (réacteur à lit circulant également appelé "slurry"). Dans ce cas, la conversion du CO peut atteindre, voire dépasser 95%. Par contre, le catalyseur mis en suspension et en circulation avec le liquide inerte, doit être séparé des produits de la réaction, puis recyclé.

Un autre procédé a été également envisagé qui consiste à opérer en présence d'une phase liquide, en mélange avec le gaz de synthèse, et d'un catalyseur en lit fixe. Ainsi le brevet US-A-4.413.063 et la demande de brevet français 95/08.637 du 13 juillet 1995 décrivent des procédés permettant de synthétiser des hydrocarbures ou des alcools à partir de gaz de synthèse en présence d'un catalyseur et d'un diluant inerte, le gaz de synthèse et la phase liquide circulant vers le bas (respectivement vers le haut) à travers le catalyseur en lit fixe. La zone réactionnelle à lit fixe avec écoulement descendant ou ascendant du liquide et du gaz, également appelé "trickle bed", est une solution qui permet d'éviter la circulation et la séparation du catalyseur tout en limitant les instabilités thermiques.

Dans le cas de la syntèse Fischer-Tropsch, l'utilisation d'un réacteur du type "trickle bed" peut toutefois conduire à une dégradation de l'activité et/ou de la sélectivité en hydrocarbures C₅⁺. Cette dégradation est en particulier très importante dans le cas d'un écoulement des fluides de haut en bas dans un réacteur travaillant à flux descendant. Dans ce cas cette dégradation est d'autant plus importante que la vitesse superficielle de la phase liquide est faible et que celle de la phase gazeuse est forte. Ce problème limite donc largement les possibilités de développement industriel d'un tel réacteur du type "trickle bed", car, pour une conversion donnée, si on veut éliminer les calories produites par la réaction et avoir des performances catalytiques suffisantes, il faudra pour pouvoir opérer à des vitesses de circulation de fluides raisonnables, des réacteurs de dimensions très importantes donc relativement chers et difficiles à mettre en oeuvre.

Il en est de même pour les autres types de réacteurs opérant en présence d'une phase liquide et d'un catalyseur en suspension (réacteurs type "slurry" ou lit bouillonnant).

Sans vouloir être lié par une quelconque théorie, il semble que cette dégradation des performances du réacteur soit due, au moins en partie, à des problèmes de distribution et de ségrégation de fluides que sont les phases gazeuse et liquide, qui entraînent qu'une partie du liquide se trouve au contact du catalyseur dans des conditions telles que la quantité de gaz de synthèse présente en cet endroit est notablement en excès ou en manque pour assurer une sélectivité et une conversion optimales à la réaction de synthèse Fischer-Tropsch.

Le problème posé est donc de trouver un moyen ou une méthode qui permette d'obtenir pour une conversion donnée de bonnes performances en termes d'activité et de sélectivité en hydrocarbures C₅⁺.

La présente invention propose une solution à ce problème permettant d'améliorer l'activité et la sélectivité de la réaction de synthèse Fischer-Tropsch.

La présente invention propose donc un moyen permettant un mélange des phases liquide et gazeuse, permettant une distribution homogène desdites phases avant le contact avec le lit de catalyseur (et de préférence avant chaque lit) dans le cas d'un réacteur de type "trickle bed" ou au sein de la phase liquide contenant le catalyseur en suspension dans le cas d'un réacteur de type "slurry" ou de type lit bouillonnant.

La présente invention concerne plus précisément un procédé de synthèse d'hydrocarbures essentiellement linéaires et saturés C₅⁺ à partir d'une phase gazeuse comprenant du gaz de synthèse, comprenant la circulation dans une zone réactionnelle d'une phase liquide et de ladite phase gazeuse, tel que la zone réactionnelle est munie d'au moins un moyen d'introduction des phases gazeuses et liquide, d'au moins un, de préférence d'un seul, moyen de soutirage de la phase hydrocarbonée formée par réaction Fischer-Tropsch, et caractérisé en ce que la zone réactionnelle comprend au moins un mélangeur statique composé de plaques disposées suivant un certain angle et qui sont empilées de manière à former des canaux ouverts se croisant, disposés obliquement par rapport à l'axe de la zone réactionnelle.

Ledit mélangeur statique est donc situé généralement en amont des, de préférence du, moyen(s) de soutirage.

Un des modes de réalisation du procédé selon l'invention est tel que au moins un moyen de mélange est aussi un moyen de distribution ou de redistribution des phases liquide et gazeuse.

Un autre des modes de réalisation du procédé selon l'invention, indépendamment ou non du mode de réalisation précédent, est tel que au moins un mélangeur statique est associé à un moyen de distribution ou de redistribution des phases liquide et gazeuse.

Un autre des modes de réalisation du procédé selon l'invention, indépendamment ou non des modes de réalisation précédents, est tel que au moins un mélangeur statique est situé dans le moyen d'introduction des phases gazeuse et liquide.

À titre d'exemple non limitatif de mélangeur statique on peut employer l'un de ceux décrits et vendus par la société SULZER et par exemple ceux connus sous la référence SMV ou SMX décrits en particulier dans la revue Chemical Engineering Progress, Vol. 75, N° 4, avril 1979 pages 61 à 65. Un autre type de mélangeur statique est également décrit dans le brevet EP-B-212 202. On trouve en outre des descriptions de mélangeur statiques utilisables dans le cadre de la présente invention dans le livre intitulé "Les réacteurs chimiques, conception, calcul et mise en oeuvre" publié par les éditions TECHNIP en 1984 en particulier pages 599 à 605.

Dans la présente invention, la phase liquide et le gaz de synthèse circulent dans une zone réactionnelle contenant soit au moins un lit catalytique fixe comportant du catalyseur sous forme de solide divisé (type "trickle bed"), soit des particules de catalyseur en suspension dans la phase liquide (type "slurry" ou lit bouillonnant).

Les particules de catalyseur ont généralement un diamètre moyen (équivalent) compris entre 0,2 et 10 mm, de manière préférée entre 0,5 à 6 mm et de manière plus préférée entre 1 et 3 mm, dans le cas d'un procédé de type "trickle bed", compris entre 30 et 300, de préférence entre 50 et 100 pm, dans le cas d'un procédé de type "slurry", et compris entre 100 et 5000, de préférence entre 0,35 et 3 mm, dans le cas d'un procédé de type lit bouillonnant.

Selon un des modes de réalisation du procédé selon l'invention, ledit procédé est de type "trickle bed". Dans un tel cas, de préférence, le procédé est tel que la zone réactionnelle comprend au moins un lit catalytique, et tel que les phases liquide et gazeuse circulent dans le même sens, et ledit procédé est caractérisé en ce que au moins un, de préférence chaque lit catalytique est associé à au moins un moyen de mélange, situé en amont dudit lit catalytique, dans le sens de la traversée du lit par lesdites phases. Alors, de façon préférée, un des modes de réalisation du procédé selon l'invention est tel que au moins un moyen de mélange situé entre deux lits catalytiques est associé en amont à un moyen de collecte des phases liquide et gazeuse et en aval à un moyen de redistribution des phases liquide et gazeuse. Un autre des modes de réalisation du procédé selon l'invention, indépendant ou non du mode de réalisation précédent, est tel que au moins un moyen de mélange situé entre deux lits catalytiques est aussi un moyen de collecte des phases liquide et gazeuse arrivant de l'amont et un moyen de redistribution des phases liquide et gazeuse allant vers l'aval. Dans le cas où le procédé est du type "trickle bed", quel que soit le mode de réalisation du procédé selon l'invention, au moins un moyen de mélange est un mélangeur statique et ledit mélangeur comprend au moins une partie, ou bien la totalité, du catalyseur présent dans le lit catalytique auquel est associé ledit mélangeur.

Dans le cas où le réacteur utilisé est de type "trickle bed", et où au moins un moyen de mélange est un mélangeur statique, plusieurs mélangeurs statiques peuvent être employés simultanément et disposés selon l'une des versions de réalisations décrites ci-devant. Lorsque le mélangeur statique ne se trouve pas immédiatement en amont du lit de catalyseur (ou du lit de particules solides non catalytiques si le lit de catalyseur est précédé d'un tel lit de particules solides) on dispose le plus souvent en amont de ce lit un moyen de distribution ou de redistribution qui consiste par exemple en un plateau de distribution classique tel que l'un de ceux décrits à la page 308 ou à la page 490 de l'ouvrage cité ci-devant. Ce plateau de distribution peut être en amont ou en aval d'un mélangeur statique. Lorsque le mélangeur statique se trouve immédiatement en amont du lit de particules solides on utilise un distributeur de fluide uniquement si ce mélangeur statique n'assure pas en même temps cette fonction de distribution.

La phase liquide ne prend pas part à la réaction et n'a aucun effet néfaste sur cette dernière. Il s'agit préférentiellement d'une coupe hydrocarbonée, de manière encore plus préférée comprenant essentiellement entre 10 et 20 atomes de carbone par molécule, telle que une coupe gasoil ou une coupe kérosène, la (ou les) fraction(s) kérosène étant constituée(s) par un mélange d'hydrocarbures dont les points d'ébullition sont approximativement compris entre 140 et 300°C, et la (ou les) fraction(s) gasoil étant constituée(s) par un mélange d'hydrocarbures dont les points d'ébullition sont approximativement compris entre 180 et 370°C lors d'une distillation atmosphérique telle que réalisée sur un brut pétrolier par l'homme du métier. Si le catalyseur est sensible au soufre, une coupe hydrocarbonée désulfurée est préférentiellement utilisée.

Dans un mode de réalisation préféré de l'invention, la phase liquide comprend au moins un produit partiellement vaporisable, par exemple entre 0 et 80% d'un produit au moins partiellement vaporisable. Ainsi les évacuations de calories et les transferts de chaleur au sein de la zone de réaction sont améliorés. On entend par 'vaporisable' tout produit liquide qui, dans les conditions de la réaction, est pratiquement complètement sous forme de gaz. On entend par 'produit partiellement vaporisable' un produit dont une partie, généralement entre 10 et 100%, est vaporisable. De façon préférée selon le présent mode de réalisation de l'invention, la phase liquide comprend une coupe hydrocarbonée partiellement vaporisable, par exemple une coupe hydrocarbonée comprenant des hydrocarbures contenant 5, 6, 7, 8, 9 ou 10 atomes de carbone par molécule.

Dans un mode de réalisation préféré selon l'invention, indépendamment ou non du mode de réalisation précédent, la phase liquide inerte est avantageusement obtenue en recyclant une partie d'une fraction des hydrocarbures produits par la réaction ; de préférence ladite fraction est la fraction gasoil ou kérosène des hydrocarbures produits par la réaction. Dans ce cas, la phase liquide inerte qui est introduite initialement dans la zone réactionnelle est apportée de l'extérieur (par opposition à une phase liquide produite dans la zone réactionnelle Fischer-Tropsch, c'est-à-dire à l'intérieur), puis ladite phase liquide comprend une partie d'une fraction des hydrocarbures produits par la réaction qui est recyclée dans ladite zone. De préférence ladite fraction est la fraction gasoil ou la fraction kérosène.

A titre indicatif, la phase liquide inerte a généralement une densité comprise entre 0,2 et 2,5 g/cm³ et une viscosité comprise entre 0,05 et 10 centipoises (0,05 à 10 mPa.s) dans les conditions de la réaction, mais ces valeurs ne sont en aucun cas obligatoires.

Dans le cas de réacteurs de type "trickle bed", le plus souvent la zone réactionnelle consiste en un réacteur allongé le long d'un axe, dont la section, qui peut être quelconque, est le plus souvent carrée, rectangulaire ou circulaire. On utilise habituellement un réacteur de section circulaire et qui comprend outre une conduite d'entrée débouchant dans le réacteur, habituellement de manière à ce que les fluides soient introduits selon la direction de l'axe dudit réacteur, une conduite de sortie habituellement orientée, au moins à proximité immédiate du réacteur, selon l'axe dudit réacteur. Les diamètres de réacteur sont généralement de l'ordre de 0,05 m à 7 m, et de préférence 0,1 à 2,5 m.

Selon une forme avantageuse de réalisation de l'invention la zone réactionnelle du procédé de type "trickle bed" consiste en un réacteur qui contient une pluralité de lits fixes contenant chacun un catalyseur pour la réaction Fischer-Tropsch, identique ou différent d'un lit à l'autre, séparés les uns des autres par au moins un moyen de mélange assurant la collecte des phases liquide et gazeuse sortant d'un lit fixe de catalyseur, le mélange des phases ainsi collectées, et la redistribution de ce mélange sur le lit fixe de catalyseur situé en aval dans le sens global de circulation desdits fluides dans ledit réacteur. Dans le cas où ledit réacteur contient une pluralité de lits catalytiques, il est particulièrement avantageux que au moins un, de préférence chaque moyen de mélange assurant la collecte des fluides, leur mélange et leur redistribution comporte en outre au moins un moyen d'introduction du gaz de synthèse dans le mélange collecté.

En restant dans cette forme de réalisation du procédé selon l'invention, le moyen de mélange situé entre deux lits de catalyseur et assurant la collecte des phases liquide et gazeuse, leur mélange et leur redistribution peut être un mélangeur statique assurant l'ensemble de ces fonctions. On ne sort cependant pas du cadre de la présente invention en utilisant un mélangeur statique n'assurant pas la fonction de redistribution des fluides et en lui adjoignant alors un distributeur de fluide classique. De façon particulièrement préférée selon l'invention, le(s) mélangeur(s) statique(s) ainsi situé(s) dans le réacteur de type "trickle bed" occupe(nt) toute la section du réacteur ou plus précisément, le mélangeur couvre une section au moins égale à celle du lit amont et/ou au moins égale à celle du lit aval (sauf pour le mélangeur situé en amont du premier lit catalytique, généralement dans la conduite d'entrée du réacteur). De cette façon, les vitesses de passage dans le(s) lit(s) et le(s) mélangeur(s) sont sensiblement identiques. Toutes combinaisons des variantes décrites sont possibles. Le procédé de la présente invention s'applique aussi bien dans le cas où la phase liquide et le gaz de synthèse circulent de haut en bas dans le réacteur, que dans le cas où l'un ou l'autre ou les deux fluides circulent de bas en haut.

Le procédé selon l'invention est particulièrement bien adapté pour être utilisé en tant que procédé de fabrication à partir d'un gaz de synthèse d'un mélange d'hydrocarbures esssentiellement linéaires et saturés, contenant généralement au moins 80% en poids, par rapport à l'ensemble des hydrocarbures formés, d'une coupe comprenant des hydrocarbures C₅⁺ et de préférence moins de 10% poids d'oléfines dans ladite coupe C₅⁺. Le procédé selon l'invention permet donc d'obtenir des hydrocarbures essentiellement paraffiniques, dont la fraction présentant les points d'ébullition les plus élevés peut être convertie avec un rendement élevé en distillats moyens (coupes gasoil et kérosène) par un procédé d'hydroconversion tel que l'hydrocraquage et/ou l'hydroisomérisation catalytique(s).

Les conditions opératoires dans lesquelles sont menées la réaction dépendent de la nature du catalyseur et sont généralement les suivantes.

La conversion du gaz de synthèse en hydrocarbures est généralement opérée sous une pression totale comprise entre 0,1 et 15 MPa, de préférence entre 0,5 et 10 MPa, la température étant comprise entre 150 et 350 °C, de préférence entre 180 et 270°C.

La vitesse volumique horaire est habituellement comprise entre 100 et 20 000 volumes de gaz de synthèse par volume de catalyseur et par heure et de préférence entre 400 et 10 000 volumes de gaz de synthèse par volume de catalyseur et par heure.

Le rapport molaire H₂/CO dans le gaz de synthèse est généralement compris entre 0,5 et 5, de préférence entre 1,2 et 3,5. La teneur en CO₂ dans le gaz de synthèse est généralement inférieure à 10 % en volume.

Dans la mise en oeuvre du procédé selon l'invention, le catalyseur est tout d'abord chargé dans la zone de réaction et préréduit par mise en contact avec au moins un composé réducteur, par exemple l'hydrogène pur ou un mélange de gaz réducteurs tels que l'hydrogène et/ou le monoxyde de carbone, et éventuellement au moins un gaz inerte tel que l'azote, le rapport molaire (composé réducteur):(composé réducteur + gaz inerte) étant compris entre 0,001:1 et 100:1 dans le cas où au moins un gaz inerte est présent. La préréduction est généralement menée entre 150 et 600° C, de préférence entre 200 et 500° C, entre 0,1 et 10 MPa, et à une vitesse volumétrique horaire de 100 à 40 000 volumes de mélange par volume de catalyseur et par heure. Cette préréduction est éventuellement menée en phase liquide, la phase liquide de la préréduction étant par exemple constituée d'au moins un hydrocarbure comprenant au moins 5 atomes de carbone par molécule.

L'invention concerne aussi un dispositif pour la mise en oeuvre du procédé tel que décrit précédemment.

Les exemples qui suivent illustrent l'invention sans toutefois en limiter la portée.

### Exemples:

Le catalyseur utilisé dans les exemples 1 à 4 est préparé de la façon suivante:

A une solution contenant 40 g de nitrate de cobalt, 1g de trichlorure de ruthénium héxamine et 0,3 g de nitrate de cuivre trihydraté dissous dans 50 ml d'eau, sont ajoutés progressivement et simultanément 75 g de solution colloïdale de silice à 40 % poids de SiO₂ (Ludox 40) et 2ml d'acide nitrique à 10%, de façon à maintenir le pH entre 1 et 2. La solution est maintenue sans agitation pendant 10mn; puis 25g de LUDOX AS40 sont ajoutés, le pH évoluant pour se stabiliser entre 5,5 et 6,5 unités pH. Après 12mn, un gel de silice contenant les sels de cobalt, cuivre, et ruthénium est formé.

Le gel obtenu est séparé des eaux mères par filtration, lavé à l'eau, séché à l'étuve entre 40 et 120°C, puis calciné à 450°C et mis en forme par pastillage. les pastilles de taille 5x5mm sont ensuite recalcinées à 600°C.

Le catalyseur est réduit dans le réacteur, préalablement à la synthèse d'hydrocarbures, au moyen d'un mélange contenant 6% d'hydrogène dans l'azote, jusqu'à 240°C, puis par de l'hydrogène pur jusqu'à 500°C, à la pression atmosphérique.

### Exemple 1 (comparatif) :

Dans cel exemple, le gaz de synthèse (mélange H2 + CO + CO₂) et la phase liquide circulent de haut en bas et passent à travers 12 dm³ de catalyseur.

Le catalyseur ci-dessus est chargé dans un réacteur de forme allongée le long d'un axe vertical, de section circulaire de diamètre 10 cm. Le gaz de synthèse utilisé pour la synthèse d'hydrocarbures consiste en un mélange contenant 66,7% d'hydrogène et 33,3% de monoxyde de carbone. Le gaz est introduit avec un débit de 12 m³/h, soit une V.V.H. gaz (vitesse volumique horaire) de 1000 h⁻¹. La réaction est opérée à 220°C et 2MPa.

La phase liquide est une coupe paraffinique C₁₀-C₁₆ ne contenant pas de soufre qui est introduite au démarrage de l'unité, puis séparée des effluents à la sortie du réacteur, et ensuite recyclée. Le débit de cette phase liquide est d'environ 200 l/h à la température de réaction, soit une vitesse spatiale de 4,5 cm/s. Les produits plus légers ou plus lourds que cette phase liquide, ainsi que l'eau coproduite dans la réaction, sont séparés, évacués et analysés.

Si l'on nomme C1, C2, C3,... Cn le nombre de molécules-grammes de monoxyde de carbone CO et de dioxyde de carbone CO₂ (si CO₂ est présent) transformées en hydrocarbures contenant de 1 à n atomes de carbone par molécule. Le nombre Nc d'atomes-grammes de carbone dans les produits formés lors de la réaction peut être calculé selon la formule suivante : Nc = C1 + 2 C2 + 3 C3 + ..... + nCn.

Alors :
- la conversion est définie comme le rapport entre le nombre Nc et le nombre de moles de CO et de CO₂ (si CO₂ est présent) dans la charge, ledit rapport étant exprimé en pourcentage
- la sélectivité en méthane CH₄ est définie comme le rapport entre le nombre C1 et le nombre Nc, ledit rapport étant exprimé en pourcentage
- la sélectivité en C₅⁺ est définie comme le rapport entre (5 C5 + 6 C6 + ... + n Cn) et Nc, ledit rapport étant exprimé en pourcentage.

Dans ces conditions, dans le présent exemple 1, la conversion est de 72 %, la sélectivité en méthane est de 8%, et la sélectivité en hydrocarbures C₅⁺ est de 86%. (voir tableau 1).

### Exemple 2 (selon l'invention):

On utilise le même réacteur que dans l'exemple 1, les mêmes conditions opératoires, et le même catalyseur en quantité identique, mais le réacteur contient deux lits de catalyseur séparés par un mélangeur statique de type SMV vendu par la société SULZER auquel est adjoint un distributeur de liquide immédiatement au dessus de ce mélangeur statique. Le mélangeur statique est formé de trois galettes successives décalées de 90 degrés d'angle l'une par rapport à l'autre comme cela est décrit dans la revue Chemical Engineering Progress, Vol. 75, N° 4, avril 1979 pages 61 à 65. Chaque galette a une hauteur de 10 cm.

Dans ces conditions, la conversion est de 78 %, la sélectivité en méthane est de 6 %, et la sélectivité en hydrocarbures C₅⁺ est de 88 % (voir tableau 1).

### Exemple 3 (selon l'invention):

On utilise le même réacteur que dans l'exemple 1, les mêmes conditions opératoires, et le même catalyseur en quantité identique, mais le réacteur contient trois lits de catalyseur séparés chacun par un mélangeur statique de type SMV vendu par la société SULZER auquel est adjoint un distributeur de liquide immédiatement au dessus de ce mélangeur statique. Chaque mélangeur statique est identique à celui employé dans l'exemple 2.

Dans ces conditions, la conversion est de 83 %, la sélectivité en méthane est de 4 %, et la sélectivité en hydrocarbures C₅⁺ est de 92 % (voir tableau 1).

### Exemple 4 (selon l'invention):

On utilise le même réacteur que dans l'exemple 1, les mêmes conditions opératoires, et le même catalyseur en quantité identique, mais la conduite d'entrée du mélange de gaz de synthèse et de la phase liquide contient un mélangeur statique de type SMV vendu par la société SULZER tel que celui décrit ci-devant dans l'exemple 2 et le réacteur contient entre le distributeur de fluide et le lit de catalyseur un mélangeur statique identique à celui inclus dans la conduite d'entrée.

Dans ces conditions, la conversion est de 81 %, la sélectivité en méthane est de 5 %, et la sélectivité en hydrocarbures C₅⁺ est de 90 % (voir tableau 1).

**Tableau 1**

| Exemples | Conversion | Sélectivité en CH₄ | Sélectivité en C₅⁺ |
|---|---|---|---|
| Ex. 1 (comparatif) 1 lit catalytique | 72 % | 8 % | 86 % |
| Ex. 2 2 lits catalytiques + 1 mélangeur | 78 % | 6 % | 88 % |
| Ex. 3 3 lits catalytiques + 2 mélangeurs | 83 % | 4 % | 92 % |
| Ex. 4 2 mélangeurs dans la conduite entrée et le réacteur | 81 % | 5 % | 90 % |

Les exemples 2 à 4 montrent que le procédé selon l'invention permet d'obtenir une meilleure sélectivité en hydrocarbures C₅⁺ et une sélectivité en méthane plus faible pour des niveaux de conversion plus élevés par comparaison avec les résultats de l'exemple 1.

Le procédé selon l'invention permet donc de
o mélanger les phases liquide(s) et gazeuse entrant dans le réacteur, lorsqu'un mélangeur est situé dans la conduite d'entrée,
o mélanger les phases liquide(s) et gazeuse en avant du lit de façon à assurer un transfert gaz/liquide pour dissoudre l'hydrogène, pour qu'il arrive au contact du solide actif une composition liquide avec un concentration importante au gaz dissous,
o remélanger les phase liquide(s) et gazeuse provenant d'un lit amont, phases qui sont plus ou moins bien séparées et qui peuvent présenter des différences de composition, avant de les mettre en contact avec un lit aval,
o mélanger le gaz de synthèse et la phase liquide injectée au niveau dudit mélangeur avec les phases liquides et gazeuses formées au cours de la réaction ou présentes dans le réacteur.
o distribuer sur toute la section du lit ledit mélange de phases de façon homogène, les concentrations de liquide et de gaz sont alors réparties uniformément sur toutes la section du lit
o et de façon annexe, d'égaliser les températures sur toute une section.

## Revendications

1. Dispositif pour la synthèse d'hydrocarbures essentiellement linéaires et saturés C₅⁺ à partir d'une phase gazeuse comprenant du gaz de synthèse, ledit dispositif comprenant au moins un moyen de circulation de la phase liquide et de la phase gazeuse dans une zone réactionnelle munie d'au moins un moyen d'introduction des phases gazeuse et liquide, d'au moins un moyen de soutirage du produit de la réaction, ledit dispositif étant **caractérisé en ce que** la zone réactionnelle comprend au moins un mélangeur statique composé de plaques disposées suivant un certain angle et qui sont empilées de manière à former des canaux ouverts se croisant, disposés obliquement par rapport à l'axe de la zone réactionnelle.

2. Dispositif selon la revendication 1 tel que la zone réactionnelle comprend au moins un lit catalytique, et tel que les phases liquide et gazeuse circulent dans le même sens, dans lequel au moins un lit catalytique est associé à au moins un mélangeur statique composé de plaques disposées suivant un certain angle et qui sont empilées de manière à former des canaux ouverts se croisant, disposés obliquement par rapport à l'axe de la zone réactionnelle, et dans lequel ledit mélangeur statique est situé en amont dudit lit catalytique, dans le sens de la traversée du lit par lesdites phases.

3. Dispositif selon la revendication 2 comprenant plusieurs lits catalytiques, **caractérisé en ce que** chaque lit catalytique est associé à au moins un desdits mélangeurs statiques.

4. Dispositif selon la revendication 3 tel que ledit mélangeur statique situé entre deux lits catalytiques est associé en amont à un moyen de collecte des phases liquide et gazeuse et en aval à un moyen de redistribution des phases liquide et gazeuse.

5. Dispositif selon la revendication 4 tel que ledit mélangeur statique situé entre deux lits catalytiques est aussi un moyen de collecte des phases liquide et gazeuse arrivant de l'amont et un moyen de redistribution des phases liquide et gazeuse allant vers l'aval.

6. Dispositif selon la revendication 5 tel que ledit mélangeur statique assurant la collecte des fluides, leur mélange et leur redistribution comporte en outre au moins un moyen d'introduction du gaz de synthèse dans le mélange collecté.

7. Dispositif selon l'une des revendications 5 à 6 tel que ledit mélangeur statique comprend au moins une partie du catalyseur présent dans le lit catalytique auquel est associé ledit mélangeur statique.

8. Dispositif selon l'une des revendications 5 à 7 tel que ledit mélangeur statique comprend la totalité du catalyseur présent dans le lit catalytique auquel est associé ledit mélangeur statique.

9. Procédé de synthèse d'hydrocarbures essentiellement linéaires saturés C₅⁺ à partir d'une phase gazeuse comprenant du gaz de synthèse, **caractérisé en ce qu'**il est mis en oeuvre en présence d'une phase liquide dans le dispositif selon l'une des revendications 1 à 8.

10. Procédé selon la revendication 9 tel que la phase liquide est une coupe hydrocarbonée.

11. Procédé selon la revendication 10 tel que ladite coupe comprend essentiellement des hydrocarbures comportant entre 10 et 20 atomes de carbone par molécule.

12. Procédé selon la revendication 9 tel que la phase liquide est une fraction gasoil ou une fraction kérosène.

13. Procédé selon l'une des revendications 9 à 12 tel que la phase liquide comprend au moins un produit partiellement vaporisable.

14. Procédé selon la revendication 9 tel que la phase liquide comprend une coupe hydrocarbonée comprenant des hydrocarbures contenant 5, 6, 7, 8, 9 ou 10 atomes de carbone par molécule.

15. Procédé selon l'une des revendications 9 à 14 tel que la conversion du gaz de synthèse en hydrocarbures est opérée sous une pression totale comprise entre 0,1 et 15 MPa, la température étant comprise entre 150 et 350 °C, la vitesse volumique horaire étant comprise entre 100 et 20 000 volumes de gaz de synthèse par volume de catalyseur et par heure, et le rapport molaire H₂/CO dans le gaz de synthèse étant compris entre 0,5 et 5.

16. Procédé selon l'une des revendications 9 à 15 tel que le catalyseur est préalablement préréduit au sein de la zone de réaction.

## Claims

1. An apparatus for the synthesis of essentially linear saturated C₅⁺ hydrocarbons from a gas phase containing synthesis gas, said apparatus comprising at least one means for circulating a liquid phase and a gas phase in a reaction zone provided with at least one means for introducing gas and liquid phases, and at least one means for extracting the reaction product, said apparatus being **characterized in that** the reaction zone also comprises at least one static mixer composed of plates disposed at a certain angle and piled up so as to form open channels which cross over, disposed obliquely with respect to the reaction zone axis.

2. Apparatus according to claim 1 wherein the reaction zone comprises at least one catalytic bed, wherein the liquid and gas phases circulate in the same direction, and wherein at least one catalytic bed is associated with at least one static mixer composed of plates disposed at a certain angle and piled up so as to form open channels which cross over, disposed obliquely with respect to the reaction zone axis, and wherein said static mixer is located upstream of said catalytic bed, in the sense of the passage of said phases over said bed.

3. Apparatus according to claim 2 comprising a plurality of catalytic beds and **characterized in that** each catalytic bed is associated with at least one mixing means.

4. Apparatus according to claim 3 wherein said static mixer located between two catalytic beds is associated upstream with a means for collecting the liquid and gas phases and downstream with a means for redistributing the liquid and gas phases.

5. Apparatus according to claim 4 wherein said static mixer located between two catalytic beds is also a means for collecting liquid and gas phases arriving from upstream and a means for redistributing the liquid and gas phases in a downstream direction.

6. Apparatus according to claim 5 wherein said static mixer for collecting, mixing and redistributing fluids further comprises at least one means for introducing synthesis gas into the collected mixture.

7. Apparatus according to claims 5 or 6 wherein said static mixer comprises at least a portion of the catalyst present in the catalytic bed with which said mixer is associated.

8. Apparatus according to any of claims 5 to 7 wherein said static mixer comprises the totality of the catalyst present in the catalytic bed with which said mixer is associated.

9. A process for the synthesis of essentially linear saturated C₅⁺ hydrocarbons from a gas phase comprising synthesis gas **characterized in that** it is operated in the presence of a liquid phase in the apparatus according to claims 1 to 8.

10. Process according to claim 9 wherein the liquid phase is a hydrocarbon cut.

11. Process according to claim 10 wherein said cut essentially comprises hydrocarbons containing 10 to 20 carbon atoms per molecule.

12. Process according to claim 9 wherein said liquid phase is a gas oil fraction or a kerosine fraction.

13. Process according to any of claims 9 to 12 wherein said liquid phase comprises at least one partially vaporisable product.

14. Process according to claim 9 wherein said liquid phase comprises a hydrocarbon cut comprising hydrocarbons containing 5, 6, 7, 8, 9 or 10 carbon atoms per molecule.

15. Process according to any one of claims 9 to 14, wherein conversion of synthesis gas to hydrocarbons is carried out at a total pressure which is in the range 0.1 to 15 MPa, a temperature which is in the range 150°C to 350°C, an hourly space velocity which is in the range 100 to 20000 volumes of synthesis gas per volume of catalyst per hour, and an H₂/CO molar ratio in the synthesis gas which is in the range 0.5 to 5.

16. Process according to any one of claims 9 to 15, wherein the catalyst is firstly reduced in the reaction zone.

## Patentansprüche

1. Vorrichtung für die Synthese von im wesentlichen linearen und gesättigten C5+-Kohlenwasserstoffen, ausgehend von einer Synthesegas umfassenden Gasphase, wobei die Vorrichtung wenigstens ein Mittel zur Zirkulation der Flüssigphase und der Gasphase in einer Reaktionszone, die mit wenigstens einem Mittel zur Einführung von gasförmigen und flüssigen Phasen ausgerüstet ist, wenigstens ein Mittel zum Abziehen des Reaktionsprodukts umfasst, wobei die Vorrichtung **dadurch gekennzeichnet ist, dass** die Reaktionszone wenigstens einen statischen Mischer umfasst, der aus Platten besteht, die gemäß einem bestimmten Winkel angeordnet sind und die derart gestapelt sind, dass offene, sich kreuzende Kanäle gebildet werden, sie schräg bezüglich der Achse der Reaktionszone angeordnet sind.

2. Vorrichtung nach Anspruch 1, derart, dass die Reaktionszone wenigstens ein katalytisches Bett umfasst und derart, dass die flüssigen und gasförmigen Phasen in gleichem Sinne zirkulieren, bei der wenigstens ein katalytisches Bett wenigstens einem statischen Mischer zugeordnet ist, der aus Platten zusammengesetzt ist, die gemäß einem bestimmten Winkel angeordnet sind und derart gestapelt sind, dass sich kreuzende offene Kanäle gebildet werden, die schräg bezüglich der Achse der Reaktionszone angeordnet sind, und bei der der statische Mischer vor dem katalytischen Bett im Sinne der Durchquerung des Betts durch diese Phasen angeordnet ist.

3. Vorrichtung nach Anspruch 2, die mehrere katalytische Betten umfasst, **dadurch gekennzeichnet, dass** jedes katalytische Bett wenigstens einem der statischen Mischer zugeordnet ist.

4. Vorrichtung nach Anspruch 3, derart, dass der statische Mischer, der zwischen zwei katalytischen Betten liegt, stromaufwärts einem Mittel zum Sammeln der flüssigen und gasförmigen Phasen und stromabwärts einem Mittel zur Verteilung der flüssigen und gasförmigen Phasen angeordnet ist.

5. Vorrichtung nach Anspruch 4, derart, dass der statische Mischer, der zwischen zwei katalytischen Betten liegt, auch ein Mittel zum Sammeln der flüssigen und gasförmigen Phasen, die aus Strömungsrichtung kommen und ein Mittel zur Verteilung der flüssigen und gasförmigen Phasen ist, die in Stromabwärtsrichtung gehen.

6. Vorrichtung nach Anspruch 5, derart, dass der statische Mischer, der das Sammeln von Fluiden, deren Mischung und deren Verteilung sicher stellt, im übrigen wenigstens ein Mittel zur Einführung von Synthesegas in das gesammelte Gemisch aufweist.

7. Vorrichtung nach einem der Ansprüche 5 bis 6, derart, dass der statische Mischer wenigstens einen Teil des Katalysators umfasst, der in dem katalytischen Bett vorliegt, welchem der statische Mischer zugeordnet ist.

8. Vorrichtung nach einem der Ansprüche 5 bis 7, derart, dass der statische Mischer die Gesamtheit des in dem katalytischen Bett vorliegenden Katalysators umfasst, welchem der statische Mischer zugeordnet ist.

9. Verfahren zur Synthese im wesentlichen linearer gesättigter C5+Kohlenwasserstoffe, ausgehend von einer gasförmigen Phase, die Synthesegas umfasst, **dadurch gekennzeichnet, dass** es in Gegenwart einer flüssigen Phase in der Vorrichtung nach einem der Ansprüche 1 bis 8 durchgeführt wird.

10. Verfahren nach Anspruch 9, derart, dass die flüssige Phase ein kohlenwasserstoffhaltiger Schnitt ist.

11. Verfahren nach Anspruch 10, derart, dass der Schnitt im wesentlichen Kohlenwasserstoffe umfasst, die zwischen 10 und 20 Kohlenstoffatome pro Molekül umfassen.

12. Verfahren nach Anspruch 9, derart, dass die flüssige Phase eine Gas-Öl-Fraktion oder eine Kerosinfraktion ist.

13. Verfahren nach einem der Ansprüche 9 bis 12, derart, dass die flüssige Phase wenigstens ein teilweise verdampfbares Produkt umfasst.

14. Verfahren nach Anspruch 9, derart, dass die flüssige Phase einen kohlenwasserstoffhaltigen Schnitt umfasst, der Kohlenwasserstoffe umfasst, die 5, 6, 7, 8, 9 oder 10 Kohlenstoffatome pro Molekül enthalten.

15. Verfahren nach einem der Ansprüche 9 bis 14, derart, dass die Umwandlung des Synthesegases zu Kohlenwasserstoffen unter einem Gesamtdruck, der zwischen 0,1 und 15 MPa liegt, einer Temperatur, die zwischen 150 und 350°C liegt, einer stündlichen Volumengeschwindigkeit, die zwischen 100 und 20000 Synthesegasvolumen pro Katalysatorvolumen und pro Stunde liegt, und einem molaren Verhältnis H₂/CO in dem Synthesegas, das zwischen 0,5 und 5 liegt, betrieben wird.

16. Verfahren nach einem der Ansprüche 9 bis 15, derart, dass der Katalysator vorher in der Reaktionszone vorreduziert wird.
